# EUROPÄISCHE PATENTANMELDUNG

(11) **EP 4 721 705 A1**
(43) Veröffentlichungstag der Anmeldung: **08.04.2026**
(21) Anmeldenummer: 24204112.7
(22) Anmeldetag: 02.10.2024
(51) Int. Cl.: A61F 2/28, A61F 2/30, A61F 2/32

(54) **SPACER ZUR GESTEUERTEN VERABREICHUNG EINES WIRKSTOFFS**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(57) **Zusammenfassung**

Die Erfindung betrifft einen implantierbaren Spacer (100) zur Abgabe eines medizinischen Fluids an einen Patienten, wobei der Spacer eine Außenoberfläche (104) und eine an der Außenoberfläche angeordnete Vielzahl von Abgabeventilen (110) aufweist, wobei die Abgabeventile jeweils durch einen innerhalb des Implantats angeordneten Kanal (101) miteinander fluidleitend verbunden sind, und wobei die Abgabeventile ausgestaltet und eingerichtet sind, sich abhängig vom Druck eines Fluids (200) innerhalb des Kanals reversibel zu öffnen, um das Fluid aus den Abgabeventilen abzugeben.

## Beschreibung

Gegenstand der Erfindung ist ein Spacer zur mehrmaligen oder kontinuierlichen lokalen Wirkstofffreisetzung. Weiterhin werden Verfahren zur lokalen Wirkstofffreisetzung beschrieben.

Unter dem Begriff "Spacer" werden üblicherweise orthopädische Implantate verstanden, die einem Patienten als temporäre Platzhalter, in der Regel nach zuvor erfolgtem Debridement des infizierten Gewebes, implantiert werden können. Spacer können der Gestalt von Hüft- und Kniegelenken oder sonstigen Gelenken nachgebildet sein oder, insbesondere zur Anwendung bei infizierten Röhrenknochen, die Gestalt von Marknägeln besitzen. Eine weitere Anwendung von Spacern ist die Beruhigung von Infektionen durch die lokale Freisetzung von antimikrobiellen Wirkstoffen im zuvor debridierten Knochen- und Weichgewebe. Herkömmliche Spacer sind meistens aus PMMA-Knochenzement gefertigt und enthalten ein Antibiotikum oder auch mehrere Antibiotika, die in das Spacer-Material eingebettet sind. Diese antimikrobiellen Wirkstoffe werden nach der Implantation aus dem PMMA-Knochenzement durch Einwirkung wässriger Körperflüssigkeiten, wie Wundsekret und Blut, herausgelöst. Die Wirkstofffreisetzung erfolgt durch Diffusionsprozesse und führt zu einer initial hohen Freisetzung und einer nachfolgenden Freisetzung geringer Wirkstoffmengen. Wünschenswerter wäre jedoch eine zeitlich konstante Freisetzung ausreichend hoher Wirkstoffmengen, um eine gleichbleibende lokale Wirkstoffkonzentrationen über einen Zeitraum von mehreren Tagen bis Wochen zu gewährleisten.

In der EP3763335B1 wurde ein Kniegelenk zur Freisetzung von Arzneimitteln beschrieben, das eine Zuleitung für Wirkstofflösungen besitzt und bei dem die Wirkstofflösung über ein Kanalsystem zu einer Vielzahl von Austrittsöffnungen gelangen kann. Problematisch ist hierbei einerseits, dass die Öffnungen, die unmittelbar in der Nähe der Zuleitung angeordnet sind, größere Flüssigkeitsvolumina freisetzen und die entfernteren Austrittsöffnungen nur geringe oder keine Flüssigkeitsvolumina freisetzen können. Weiterhin kann koaguliertes Blut oder einwachsendes Bindegewebe die Austrittsöffnungen verstopfen.

Ein ähnliches Kniegelenkspacersystem wurde in der Patentschrift US10864314B2 offenbart.

In der WO2016205077A wurden Spacer mit einer Spülfunktion beschrieben, wobei die Spülflüssigkeit an der Spaceroberfläche durch Rillen an der Spaceroberfläche geleitet wird.

In der EP3542759B1 wird ein ähnliches Spacersystem vorgeschlagen, das Austrittsöffnungen und auch Öffnungen zum Ableiten der Spülflüssigkeit besitzt.

Es wurden weiterhin Marknägel offenbart, die zur Beruhigung von Infektionen vorgesehen sind. Diese Marknägel besitzen eine durch Öffnungen perforierte Wand. Durch einen angeschlossenen Schlauch können von außerhalb des Patienten antibiotische oder antiseptische Lösungen in die Marknägel eingeleitet werden (US5681289A, CN2857862Y, CN201370624Y, WO2016205077A1). Die antibiotischen oder antiseptischen Lösungen treten durch Öffnungen in der Marknagelwand aus. Problematisch an diesen Konzepten ist, dass die Öffnungen durch koaguliertes Blut verstopfen können und dass die Öffnungen nach wenigen Tagen durch einwachsendes Bindegewebe teilweise oder auch vollständig verschlossen werden können. Weiterhin ist es problematisch, dass antibiotische oder antiseptische Lösungen bevorzugt aus den Öffnungen des Marknagels austreten, die sich unmittelbar neben dem Anschluss des Schlauchs befinden. Durch weiter vom einleitenden Schlauch entfernte Öffnungen werden nur noch kleine Volumina der antibiotischen oder antiseptischen Lösungen freigesetzt, weil die Lösung auf dem kürzesten Weg hinter dem einleitenden Schlauch aus den Marknägel austritt. Eine Freisetzung konstanter Volumina der antibiotischen oder antiseptischen Lösungen über die gesamte Länge der Marknägel ist daher nicht gewährleistet.

### BEVORZUGTE AUSFÜHRUNGSFORMEN

Eine Aufgabe der vorliegenden Erfindung besteht darin, eines oder mehrere der oben geschilderten und weitere Probleme des Stands der Technik zu lösen.

Der vorliegenden Erfindung liegt insbesondere die Aufgabe zu Grunde, einen Spacer bereitzustellen, der eine mehrmalige oder eine kontinuierliche Wirkstofffreisetzung an der Außenoberfläche des Spacers über mehrere Tage bis zu Wochen ermöglicht. Ein medizinisches Fluid, wie z.B. wässrige Wirkstofflösungen, kann hierzu beispielsweise über eine Zuleitung in den Spacer von außen eingeleitet werden und das Fluid anschließend aus mehreren Abgabeventilen an der Außenoberfläche des Spacers austreten. Der erfindungsgemäße Spacer ist bevorzugt so gestaltet, dass eine Freisetzung annähernd gleicher Volumina der Wirkstofflösung gleichzeitig aus allen Abgabeventilen erfolgen kann. Weiterhin kann bevorzugt ein Verstopfen der Abgabeventilen durch koaguliertes Blut und durch einwachsendes Bindegewebe vermieden werden. Erwünscht ist weiterhin, dass Flüssigkeiten von außen nicht durch die Abgabeventile in das Innere des Spacers dringen können. Die Abgabeventile können so gestaltet sein, dass diese nicht über die Außenoberfläche des Spacers herausragen und dass während der Abgabe eines medizinischen Fluids keine Komponenten der Spacer in das umgebende Gewebe gedrückt werden. Der Spacer kann eine Abgabe relativ geringer Volumina eines medizinischen Fluids, beispielsweise im Bereich bis maximal 50 ml pro einmaliger Applikation, ermöglichen. Der Spacer kann weiterhin zur Abgabe eines medizinischen Fluids mit einer hohen Wirkstoffkonzentration geeignet sein. Bevorzugt eignet sich der erfindungsgemäße Spacer zur präzise gesteuerten Abgabe von Wirkstoffen, wobei bevorzugt an verschiedenen Stellen des Spacers eine gleichmäßige Abgabe erfolgen kann. Dies ist ein Vorteil gegenüber herkömmlichen Spacer-Systemen, welche eine Spülfunktion mit der Einleitung größerer Volumina in den Spacer zur Spülung des umgebenden Gewebes bezwecken. Erfindungsgemäß benötigt der hierin beschriebene Spacer bevorzugt keine weiteren Elemente zur Ableitung oder Wiederaufnahme einer Spülflüssigkeit. Der Spacer kann zur temporären Implantation in zuvor infizierte und debridierte Knochenkavitäten geeignet sein.

Diese Aufgaben werden gelöst durch die hierin beschriebenen Verfahren, Vorrichtungen, Kits und medizinischen Verwendungen, insbesondere denjenigen, die in den Patentansprüchen beschrieben sind.

Bevorzugte Ausführungsformen der Erfindung werden nachfolgend beschrieben.

Eine erste Ausführungsform eines ersten Aspekts der Erfindung betrifft einen implantierbaren Spacer zur Abgabe eines medizinischen Fluids an einen Patienten, wobei der Spacer eine Außenoberfläche und eine an der Außenoberfläche angeordnete Vielzahl von Abgabeventilen aufweist, wobei die Abgabeventile jeweils durch einen innerhalb des Implantats angeordneten Kanal miteinander fluidleitend verbunden sind, und wobei die Abgabeventile ausgestaltet und eingerichtet sind, sich abhängig vom Druck eines Fluids innerhalb des Kanals reversibel zu öffnen, um das Fluid aus den Abgabeventilen abzugeben.

Eine zweite Ausführungsform der Erfindung betrifft einen Spacer gemäß der ersten Ausführungsform, wobei die Abgabeventile jeweils ein hülsenförmiges Gehäuse aufweisen, und wobei der Spacer eine Vielzahl von Aufnahmen aufweist, wobei die Abgabeventile durch formschlüssiges und/oder kraftschlüssiges Eingreifen der Gehäuse in die Aufnahmen mit dem Spacer verbindbar sind.

Eine dritte Ausführungsform der Erfindung betrifft einen Spacer nach einer der vorangehenden Ausführungsformen, wobei die Abgabeventile jeweils ein erstes Material aufweisen, wobei das erste Material gummielastisch ist, und das erste Material weiterhin jeweils einen Schlitz aufweist.

Eine vierte Ausführungsform der Erfindung betrifft einen implantierbaren Spacer gemäß der dritten Ausführungsform, wobei die Schlitze eingerichtet sind, sich durch eine elastische Rückstellkraft des ersten Materials reversibel zu öffnen und schließen.

Eine fünfte Ausführungsform der Erfindung betrifft einen implantierbaren Spacer gemäß der dritten oder vierten Ausführungsform, wobei das erste Material eine Shore-A-Härte von 30 bis 80, bevorzugt 40 bis 70, weiter bevorzugt 50 bis 60, aufweist.

Eine sechste Ausführungsform der Erfindung betrifft einen implantierbaren Spacer nach einer der vorangehenden Ausführungsformen, wobei der Kanal eine Einlassöffnung zur Aufnahme eines Fluids aufweist, wobei die Einlassöffnung bevorzugt an der Außenoberfläche des Spacers angeordnet ist.

Eine siebte Ausführungsform der Erfindung betrifft einen implantierbaren Spacer gemäß der zweiten und sechsten Ausführungsform, wobei die Einlassöffnung in identischer Weise wie die Aufnahmen ausgestaltet ist, oder wobei die Einlassöffnung in anderer Weise als die Aufnahmen ausgestaltet ist.

Eine achte Ausführungsform der Erfindung betrifft einen implantierbaren Spacer nach einer der vorangehenden Ausführungsformen, wobei der Spacer aus der Gruppe bestehend aus einem Kniegelenkspacer, einem Hüftgelenkspacer, einem Wirbelkörperspacer und einem Marknagelspacer ausgewählt ist.

Eine neunte Ausführungsform der Erfindung betrifft einen implantierbaren Spacer nach einer der vorangehenden Ausführungsformen, wobei der Spacer ausgebildet und eingerichtet ist, aus jedem der Abgabeventile gleichzeitig eine im Wesentlichen identische Menge eines Fluids abzugeben.

Eine zehnte Ausführungsform der Erfindung betrifft einen implantierbaren Spacer nach einer der vorangehenden Ausführungsformen, wobei der Spacer ausgebildet und eingerichtet ist, alle Abgabeventile jeweils gleichzeitig zu öffnen, und/oder jeweils gleichzeitig zu schließen.

Eine elfte Ausführungsform der Erfindung betrifft einen implantierbaren Spacer nach einer der vorangehenden Ausführungsformen, wobei die Abgabeventile jeweils derart angeordnet sind, dass sie mit der Außenoberfläche des Spacers abschließen.

Eine zwölfte Ausführungsform der Erfindung betrifft einen implantierbaren Spacer nach einer der vorangehenden Ausführungsformen, wobei der Kanal mehrere Verzweigungen aufweist, und wobei bevorzugt jede Verzweigung ein Abgabeventil oder eine Einlassöffnung gemäß der sechsten Ausführungsform aufweist.

Eine dreizehnte Ausführungsform betrifft einen implantierbaren Spacer nach einer der vorangehenden Ausführungsformen, wobei die Abgabeventile ausgestaltet und eingerichtet sind, Flüssigkeit nur unidirektional durch die Abgabeventile passieren zu lassen.

Ein zweiter Aspekt der Erfindung betrifft ein medizinisches Fluid zur Anwendung in einem medizinischen Verfahren, wobei das Verfahren die nachfolgenden Schritte aufweist: Bereitstellen eines implantierbaren Spacers nach einer der vorangehenden Ausführungsformen, Einbringen eines medizinischen Fluids in den Kanal des Spacers,
Druckbeaufschlagung des medizinischen Fluids in dem Kanal, um die Abgabeventile druckabhängig zu öffnen und das Fluid an einen Patienten abzugeben.

Eine weitere Ausführungsform gemäß des zweiten Aspekts der Erfindung betrifft ein Fluid zur Anwendung gemäß der vorangehenden Ausführungsform, wobei das Fluid einen Wirkstoff aufweist, der ausgewählt ist aus der Gruppe bestehend aus einem Antibiotikum, einem Antiphlogistikum, einem Anästhetikum und einem Cytostatikum.

### KURZE BESCHREIBUNG DER ZEICHNUNGEN

**Figur** 1 zeigt einen erfindungsgemäßen Spacer, der als Hüftgelenkspacer ausgestaltet ist.
**Figur** 2 zeigt einen erfindungsgemäßen Spacer, welcher mit einem medizinischen Fluid befüllt ist.
**Figur** 3 zeigt eine Ausführungsform eines Abgabeventils.
**Figur 4** zeigt einen Ausschnitt eines erfindungsgemäßen Spacers mit einem modularen Abgabeventil.
**Figur 5** zeigt ein Abgabeventil in einem vollständig geöffneten Zustand.
**Figur 6** zeigt ein Abgabeventil in einem teilweise geöffneten Zustand.
**Figur 7** zeigt ein Abgabeventil in einem geschlossenen Zustand.
**Figur 8** zeigt einen erfindungsgemäßen Spacer, der als Kniegelenkspacer ausgestaltet ist.
**Figur 9** zeigt einen erfindungsgemäßen Kniegelenkspacer mit Gelenkelementen.
**Figur 10** zeigt einen erfindungsgemäßen Spacer, welcher als Wirbelkörperspacer ausgestaltet ist.
**Figur 11** zeigt einen erfindungsgemäßen Spacer, welcher als Marknagelspacer ausgebildet ist.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

"Operativ verbunden" oder "operativ verbindbar", bedeutet hierin, dass zwei betreffende Elemente eine funktionelle Beziehung zueinander aufweisen. Beispielsweise kann ein erstes Element eingerichtet sein, ein zweites Element durch eine solche operative Verbindung zu steuern oder zu bewegen. Der Begriff "steuern" umfasst hierin auch die Sperrung oder Freigabe einer Funktion, beispielsweise das Ermöglichen oder die Einschränkung der Bewegung oder sonstigen Funktion eines Elements.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse, Vorrichtungen, Kits und Verwendungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Die hierin beschriebenen technischen Normen und Standards, beispielsweise im Zusammenhang mit Testverfahren, beziehen sich jeweils auf die zum Prioritätsdatum der vorliegenden Anmeldung aktuelle Fassung.

Ein Aspekt der Erfindung betrifft einen implantierbaren Spacer zur Abgabe eines medizinischen Fluids an einen Patienten, wobei der Spacer eine Außenoberfläche und eine an der Außenoberfläche angeordnete Vielzahl von Abgabeventilen aufweist, wobei die Abgabeventile jeweils durch einen innerhalb des Implantats angeordneten Kanal miteinander fluidleitend verbunden sind, und wobei die Abgabeventile ausgestaltet und eingerichtet sind, sich abhängig vom Druck eines Fluids innerhalb des Kanals reversibel zu öffnen, um das Fluid aus den Abgabeventilen abzugeben.

Der Begriff "Spacer" bezeichnet hierin ein medizinisches Implantat, welches dazu ausgebildet und eingerichtet ist, als temporärer Platzhalter anstelle eines Knochens oder Gelenks, oder eines Teils davon, einem Patienten implantiert zu werden. In einer Ausführungsform umfasst der Spacer ein biokompatibles Material, wie z.B. PMMA, Edelstahl oder Titan. Der Spacer kann ein Metall, einen Kunststoff oder ein Metall-Kunststoff-Komposit umfassen, oder daraus bestehen. Beispiele für biokompatible Metalle sind 316L-Stahl, Cobalt-Chrom-Stahl, Titan, und Titanlegierungen. Beispiele für biokompatible Kunststoffe sind Polymethylmethacrylat, Polyamid 12, Polyethersulfon und Polyetherketon. In einer Ausführungsform besteht der Spacer zu mindestens 90% (Masse/Masse) aus PMMA.

In einer Ausführungsform ist der erfindungsgemäße Spacer durch SLM (Selective Laser Melting) oder durch EBM (Electron Beam Melting) aus Edelstahl oder Titan oder anderen biokompatiblen Metallen oder Legierungen herstellbar oder hergestellt. In einer Ausführungsform ist der erfindungsgemäße Spacer durch SLS (Selektives Laser Sintern) aus hierfür geeigneten Kunststoffen, wie Polyamid 12 oder Polymethylmethacrylat herstellbar oder hergestellt. In einer Ausführungsform ist der erfindungsgemäße Spacer durch Kunststoffspritzgießen aus thermoplastischen Kunststoffen herstellbar oder hergestellt. Dabei kann der Spacer aus mehreren spritzgegossenen Teilen zusammengefügt sein, wobei diese Teile beispielsweise durch Verschweißen oder Verkleben miteinander verbunden sein können.

Der hierin beschriebene Spacer ist bevorzugt zur Abgabe eines medizinischen Fluids ausgebildet und eingerichtet. Ein "medizinisches Fluid" bezeichnet hierin ein Fluid, welches zur medizinischen Anwendung dient, und eine medizinische Wirkung aufweist.

Unter dem Begriff "medizinisches Fluid" werden hierin insbesondere wässrige und nichtwässrige Flüssigkeiten verstanden, die gelöste Wirkstoffe, insbesondere pharmazeutische Wirkstoffe, enthalten können, oder selbst eine medizinische Wirkung haben können. Weiterhin fallen unter diesen Begriff auch Gase und Gas-Flüssigkeitsgemische, die eine pharmakologische Wirkung im humanen oder tierischen Organismus entfalten können. In einer Ausführungsform weist das medizinische Fluid einen Wirkstoff auf. In einer Ausführungsform ist der Wirkstoff ausgewählt aus der Gruppe bestehend aus einem Antibiotikum, einem Antimykotikum, einem Cytostatikum, einem Anaesthetikum, einem osteoinduktiven Wirkstoff, und einem Antiphlogistikum.

In einer Ausführungsform ist der Wirkstoff ein Antibiotikum. In einer Ausführungsform ist das Antibiotikum ausgewählt aus der Gruppe bestehend aus Penicillinen, Cephalosporinen, Carbapenemen, Chinolonen, Makroliden, Lincosamiden, Aminoglycosiden und Glycopeptiden. Beispiele für Penicilline sind Amoxicillin und Benzylpenicillin. Beispiele für Cephalosporine sind Ceftriaxon und Cefuroxim. Beispiele für Carbapeneme sind Meropenem und Imipenem. Beispiele für Chinolone sind Ciprofloxacin und Levofloxacin. Beispiele für Makrolide sind Azithromycin und Clarithromycin. Beispiele für Glycopeptide sind Vancomycin und Teicoplanin. Beispiele für Aminoglykoside sind Gentamicin und Tobramycin. Ein Beispiel für ein Aminoglycosid ist Clindamycin.

In einer Ausführungsform ist der Wirkstoff ein Antimykotikum. Beispiele für Antimykotika umfassen Polyene (z.B. Amphotericin B, Nystatin, Natamycin), Azole (z.B. Fluconazol, Voriconazol), Echinocandine (z.B. Caspofungin, Micafungin), und Allylamine (z.B. Terbinafin). In einer Ausführungsform ist der Wirkstoff ein Cytostatikum. Beispiele für Cytostatika umfassen alkylierende Substanzen, Antimetabolite, Naturstoffe, Proteinkinase-Inhibitoren und monoklonale Antikörper. Beispiele für alkylierende Substanzen umfassen Cyclophosphamid, Melphalan und Busulfan. Beispiele für Antimetabolite umfassen Methotrexat, 5-Fluorouracil und Gemcitabin. Beispiele für Naturstoffe umfassen Paclitaxel, Doxorubicin und Vincristin. Beispiele für Proteinkinase-Inhibitoren umfassen Imatinib, Gefitinib und Sunitinib. Beispiele für monoklonale Antikörper umfassen Rituximab, Trastuzumab und Bevacizumab.

In einer Ausführungsform ist der Wirkstoff ein Anästhetikum. Beispiele für Anästhetika umfassen Lidocain, Bupivacain, Ropivacain, Propofol, Etomidat, Ketamin, Morphin, Fentanyl, und Remifentanyl.

In einer weiteren Ausführungsform ist der Wirkstoff ein osteoinduktiver Wirkstoff. Beispiele für osteoinduktive Wirkstoffe umfassen Knochenmorphogenetische Proteine (BMPs), Parathormonverwandte Peptide, Anti-Sklerostin-Antikörper und Wachstumsfaktoren. Beispiele für Knochenmorphogenetische Proteine umfassen BMP-2 und BMP-7. Ein Beispiel für ein Parathormon-verwandtes Peptid ist Teriparatid (PTH 1-34). Ein Beispiel für einen Anti-Sklerostin-Antikörper ist Romosozumab. Beispiele für Wachstumsfaktoren umfassen Fibroblasten-Wachstumsfaktoren (FGFs) und Platelet Derived Growth Factor (PDGF).

In einer weiteren Ausführungsform ist der Wirkstoff ein Antiphlogistikum. Beispiele für Antiphlogistika umfassen nichtsteroidale Antirheumatika (NSAR), Glukokortikoide, selektive COX-2-Inhibitoren, Biologika (z.B. TNF-α-Inhibitoren) und Januskinase-Inhibitoren. Beispiele für nichtsteroidale Antirheumatika (NSAR) umfassen Ibuprofen, Diclofenac und Naproxen. Beispiele für Glukokortikoide umfassen Prednison, Dexamethason und Hydrocortison. Beispiele für selektive COX-2-Inhibitoren umfassen Celecoxib und Etoricoxib. Beispiele für TNF-α-Inhibitoren umfassen Infliximab, Adalimumab und Etanercept. Beispiele für Januskinase-Inhibitoren umfassen Tofacitinib und Baricitinib.

In einer Ausführungsform ist der Wirkstoff geeignet zur Behandlung einer Knochenerkrankung. In einer Ausführungsform ist der Wirkstoff ausgewählt aus der Gruppe aus Bisphosphonaten (z.B. Alendronat, Zoledronat), Kalzitonin, selektiven Östrogen-Rezeptor-Modulatoren (z.B. Raloxifen) und Strontiumranelat. In einer Ausführungsform umfasst der Wirkstoff Hyaluronsäure oder ein Kortikosteroid (z.B. Betamethason, Triamcinolon). In einer Ausführungsform umfasst der Wirkstoff ein Calciumsalz. Beispiele für geeignete Calciumsalze umfassen Calciumphosphate und Calciumsulfate. Beispiele für Calciumphosphate umfassen Beta-TCP und Hydroxylapatit.

In einer Ausführungsform ist der Wirkstoff ausgewählt aus der Gruppe bestehend aus Gentamicin, Tobramycin, Amikacin, Clindamycin, Daptomycin, Vancomycin, Teicoplanin, Dalbavancin, Fosfomycin, Linezolid, Eperezolid, Colistin, Meropenem, Fluconazol, Micafungin, Caspofungin, Metronidazol, Moxifloxazin, Ofloxazin, Levofloxacin, Ciprofloxazin, Rifamycin und Rifampicin.

In einer Ausführungsform umfasst das medizinische Fluid eine wässrige Lösung eines hierin beschriebenen Wirkstoffs.

Der Spacer weist eine Außenoberfläche auf. An dieser Außenoberfläche sind mehrere Abgabeventile angeordnet. Die Abgabeventile sind zur Abgabe eines medizinischen Fluids ausgebildet und eingerichtet. Die Abgabeventile sind untereinander über einen Kanal verbunden. Der Kanal ist in einem Innenraum des Spacers angeordnet. In einigen Ausführungsformen weist der Spacer eine Einlassöffnung auf. Die Einlassöffnung ist bevorzugt an der Außenoberfläche des Spacers angeordnet. Die Einlassöffnung ist zum Einbringen eines medizinischen Fluids in den Kanal ausgebildet und eingerichtet. Der Kanal verbindet in einer Ausführungsform, welche eine Einlassöffnung und mehrere Abgabeventile aufweist, die Abgabeventile sowohl untereinander als auch mit der Einlassöffnung.

Die Abgabeventile sind bevorzugt ausgebildet und eingerichtet, sich abhängig vom Druck eines Fluids innerhalb des Kanals reversibel zu öffnen, um das Fluid aus den Abgabeventilen abzugeben. Hierdurch wird eine gesteuerte Abgabe des Fluids aus dem Spacer ermöglicht.

In einigen Ausführungsformen sind die Abgabeventile im Wesentlichen gleichmäßig über die gesamte Außenoberfläche des Spacers verteilt angeordnet, sodass ein Fluid gleichmäßig an die gesamte Umgebung des Spacers abgegeben werden kann. Hierbei kann eine Abgabe des Fluids in alle Raumrichtungen erfolgen.

Zur gleichmäßigen Abgabe eines medizinischen Fluids an die Umgebung des Spacers kann es weiterhin vorteilhaft sein, im Verhältnis zur Oberfläche des Spacers eine hierfür ausreichende Menge von Abgabeventilen vorzusehen. In einer Ausführungsform umfasst der Spacer daher mindestens ein Abgabeventil pro 16,0 cm² Außenoberfläche des Spacers, weiterhin bevorzugt mindestens ein Abgabeventil pro 9,0 cm² Außenoberfläche des Spacers.

Die Abgabeventile können mit dem Material des Spacers eine Einheit bilden, d.h. in den Spacer integriert sein. Beispiele hierfür umfassen die hierin beschriebenen Schlitzventile, wobei beispielsweise Öffnungen an der Außenoberfläche des Spacers mit einem gummielastischem Material beschichtet und überspannt sind. Ein solches gummielastisches Material, das zur Herstellung von Schlitzventilen geeignet ist, wird hierin auch als "erstes Material" bezeichnet.

Die Abgabeventile können modular aufgebaut sein, sodass der übrige Teil des Spacers getrennt von den Abgabeventilen hergestellt und anschließend die Ventile mit dem übrigen Teil des Spacers zusammengefügt werden können. Dies erlaubt eine flexible und kostengünstige Herstellung, da beispielsweise gleichartige, modulare Abgabeventile in unterschiedlich geformten Spacern verwendet werden können.

In einigen Ausführungsformen weisen die Abgabeventile jeweils ein hülsenförmiges Gehäuse auf. In einigen Ausführungsformen weist der Spacer eine Vielzahl von Aufnahmen auf, die ein Zusammenfügen solcher Abgabeventile mit dem Spacer ermöglichen. In einigen Ausführungsformen sind die Abgabeventile durch formschlüssiges und/oder kraftschlüssiges Eingreifen der Gehäuse in die Aufnahmen mit dem Spacer verbindbar oder verbunden.

Das Abgabeventil ist bevorzugt ein Überdruckventil, d.h. ein Ventil, das sich oberhalb eines definierten Drucks öffnet. Dieser Druck wird hierin als "Grenzdruck" bezeichnet. Bevorzugt weisen alle Abgabeventile des Spacers denselben Grenzdruck auf. Weiterhin sind Ventile bevorzugt, die in Bezug auf die Umgebung des Spacers "berührungslos" sind, d.h. derart ausgestaltet sind, bei Ihrer Öffnung keine Teile in Richtung des umliegenden Gewebes zu bewegen. Hiermit kann eine Reizung des empfindlichen Patientengewebes, insbesondere bei vorliegenden Entzündungen, vermieden werden. Beispiel für solche Abgabeventile sind solche, die eine gummielastische Membran mit einer darin angeordneten schlitzförmigen Öffnung aufweisen, wie nachfolgend beschrieben.

In einer Ausführungsform sind die Abgabeventile ausgebildet und eingerichtet, sich oberhalb eines Grenzdrucks eines Fluids innerhalb des Kanals zu öffnen, welcher 1 bar (10^5 Pa), höher als der Druck außerhalb des Spacers beträgt, und sich unterhalb dieses Grenzdrucks fluiddicht zu schließen. Dies bedeutet, dass die Abgabeventile geschlossen sind, wenn der Überdruck eines Fluids in dem Kanal geringer als 1 bar ist, und die Abgabeventile geöffnet sind, wenn der Überdruck eines Fluids in dem Kanal 1 bar oder höher ist. Mit "Überdruck" ist hierbei die Differenz zwischen dem atmosphärischen Druck und dem Druck des Fluids in dem Kanal des Spacers bezeichnet.

In einer Ausführungsform weisen die Abgabeventile jeweils ein erstes Material auf. Das erste Material ist gummielastisch. Ein gummielastisches Material zeichnet sich insbesondere dadurch aus, dass es nach einer mechanischen Verformung selbstständig seine ursprüngliche Form wieder einnimmt. Erfindungsgemäß verhilft diese Eigenschaft, wie nachfolgend weiter ausgeführt, einem in einem im elastischen Material angeordneten Schlitz die Fähigkeit, sich Druck abhängig zu öffnen bzw. zu schließen.

Die Abgabeventile können beispielsweise ein erstes Material aufweisen, welches in Form einer Beschichtung der Außenoberfläche des Spacers ausgestaltet ist. Der Kanal des Spacers kann in eine Öffnung an der Außenoberfläche des Spacers münden, wobei diese Öffnung mit einer solchen Beschichtung versehen sein kann. Hierbei kann die Beschichtung eine dichtende Begrenzung einer solchen Öffnung bilden.

In einer Ausführungsform weisen die Abgabeventile jeweils ein Gehäuse auf, in welches ein solches erstes Material eingebracht ist. Beispielsweise kann das Gehäuse eine im wesentlichen becherförmige Geometrie aufweisen, welche eine Öffnung aufweist. Das erste Material kann in einer solchen Ausführungsform die Öffnung in dem Gehäuse des Abgabeventils fluiddicht verschließen. Dabei kann das erste Material scheibenförmig oder becherförmig ausgestaltet sein.

Die Abgabeventile können formschlüssig, kraftschlüssig und/oder stoffschlüssig mit dem Spacer verbindbar sein. Beispielsweise können die Abgabeventile durch Verpressen und/oder Verkleben und/oder Verschweißen und/oder Verschrauben mit dem Spacer verbindbar oder verbunden sein. Insbesondere kann eine solche Verbindung zwischen einem Gehäuse eines Abgabeventils und einer hierin beschriebenen Aufnahme des Spacers bestehen.

In einer Ausführungsform weist das erste Material eine Shore-A-Härte im Bereich von 30 bis 80 auf. In einer Ausführungsform weist das erste Material einer Shore-A-Härte von 40 bis 70, oder 50 bis 60, beispielsweise ungefähr 55, auf. Die Shore-A-Härte wird nach ASTM D2240 bestimmt. Elastomere in diesem Bereich der Shore A-Härte haben eine sehr gute Rückstellkraft und eignen sich sehr gut zur Herstellung einer hierin beschriebenen gummielastischen Scheibe oder gummielastischen Beschichtung als Teil eines Abgabeventils. Schlitze in solchen Elastomeren verschließen sich bei Abfall des darauf einwirkenden Drucks selbstständig und schnell. In einer Ausführungsform weist das erste Material eine Shore-A-Härte im Bereich von 75 bis 95 auf.

Das erste Material ist bevorzugt ein Polymer, insbesondere ein Elastomer. Bevorzugt umfasst das erste Material ein medizinisch verträgliches Elastomer, oder besteht daraus. Beispiele für medizinisch verträgliche Elastomere umfassen Silikonelastomere, thermoplastische Elastomere (TPEs), Polyisopren, Butylkautschuk, Nitrilkautschuk, Ethylen-Propylen-Dien-Monomer (EPDM), Chloropren-Kautschuk, Fluroelastomere, Perfluorelastomere und Polyacrylatelastomere. Beispiele für Silikonelastomere umfassen Polydimethylsiloxan (PDMS) und flüssiger Silikonkautschuk (LSR).

Beispiele für thermoplastische Elastomere (TPEs) umfassen Styrol-Block-Copolymere (SBCs) wie Styrol-Ethylen-Butylen-Styrol (SEBS), thermoplastische Polyurethane (TPU) und thermoplastische Copolyester (TCE). Beispiele für Polyisopren umfassen natürlichen Gummi und synthetischen Polyisopren. Beispiele für Butylkautschuk umfassen Brombutylkautschuk (BIIR) und Chlorbutylkautschuk (CIIR). Ein bevorzugtes Polyurethan ist ein Polyetherurethan. Polyetherurethane sind durch Polyadditionsreaktion eines Polyetherpolyols mit einem Diisocyanat herstellbar.

In einer Ausführungsform weist das erste Material ein thermoplastisches Elastomer auf. In einer Ausführungsform besteht das erste Material aus einem thermoplastischen Elastomer. In einer Ausführungsform weist das erste Material ein Polyetherurethan oder Ethylen-Propylen-dienKautschuk auf. In einer Ausführungsform umfasst das erste Material ein Polyetherurethan. In einer Ausführungsform besteht das erste Material aus Polyetherurethan.

In einer Ausführungsform weist das erste Material nur ein einziges Elastomer auf, d. h. dem ersten Material ist kein zweites Elastomer beigemischt. In einer Ausführungsform weist das erste Material mindestens zwei unterschiedliche Materialien, beispielsweise zwei unterschiedliche Elastomere, auf. Hierdurch kann beispielsweise die Härte des ersten Materials auf einen gewünschten Zielwert eingestellt werden. Das erste Material kann ein Copolymer aufweisen. Das Copolymer kann ein thermoplastisches Elastomer sein. Bevorzugt sind Copolymere, welche ein weiches Segment und ein hartes Segment in ihrer Molekülkette aufweisen. Durch das Verhältnis zwischen dem weichen Segment und dem harten Segment innerhalb der Molekülkette des Copolymers können die physikalischen Eigenschaften eines solchen Copolymers eingestellt werden, beispielsweise die Shore-A-Härte des Copolymers. Das harte Segment kann mithilfe eines Verbinders (Linker) mit dem weichen Segment verbunden sein.

Weiterhin kann das erste Material ein Additiv zur Einstellung der Härte aufweisen. Beispiele für solche Additive sind Füllstoffe und Weichmacher. Beispiele für Füllstoffe umfassen Kieselsäuren, Titandioxid, Calciumcarbonat, Bariumsulfat und Kohlenstoff.

Beispiele für Weichmacher umfassen Adipate, Trimellitate, citratbasierte Weichmacher und Ester von mehrwertigen Alkoholen.

Beispielsweise können als Weichmacher TOTM (Tris(2-ethylhexyl)trimellitat), DINCH (Diisononylcyclohexan-1,2-dicarboxylat), ATBC (Acetyltributylcitrat), oder DEHA (Di(2-ethylhexyl)adipat) verwendet werden.

In einer Ausführungsform ist das erste Material frei von Weichmachern. In einer Ausführungsform ist das erste Material frei von Füllstoffen. In einer Ausführungsform ist das erste Material frei von endokrin wirkenden Substanzen wie z.B. Phtalaten oder Bisphenolen.

In einer Ausführungsform kann das erste Material weiterhin ein Gleitmittel aufweisen. Bevorzugt ist das Gleitmittel medizinisch verträglich. Bevorzugt ist das Gleitmittel frei von polyhalogenierten Substanzen und Silikonen. In einer Ausführungsform weist das Gleitmittel einen Naturstoff auf, beispielsweise ein Lipid, ein Triglycerid oder ein Biopolymer.

Das erste Material kann in einer Ausführungsform einen Young-Elastizitätsmodul von 1,2 × 10^7 Pa bis 2,1 x × 10^7 Pa aufweisen, beispielsweise 1,3 × 10^7 Pa bis 2,0 x × 10^7 Pa, 1,4 × 10^7 Pa bis 1,9 x × 10^7 Pa, 1,5 × 10^7 Pa bis 1,8 x × 10^7 Pa, oder 1,5 × 10^7 Pa bis 1,7 x × 10^7 Pa. In einer Ausführungsform kann das erste Material einen Young-Elastizitätsmodul von ungefähr 1,6 × 10^7 Pa aufweisen. In einer Ausführungsform kann das erste Material einen Young-Elastizitätsmodul von 2000 bis 2500 psi aufweisen. Letzteres entspricht ungefähr 1,4× 10^7 Pa bis 1,7 × 10^7 Pa.

Der Young-Elastizitätsmodul kann nach ASTM D412 bestimmt werden.

Das erste Material ist bevorzugt mithilfe gängiger Sterilisationsverfahren sterilisierbar, d. h. im Rahmen dieser Verfahren beständig gegenüber UV-Strahlung, Gammastrahlung und Behandlung mit Ethylenoxid.

Das erste Material ist bevorzugt mithilfe fachüblicher Extrusionsverfahren und/oder Spritzgussverfahren formbar.

Das erste Material kann jeweils einen Schlitz aufweisen. In einer Ausführungsform weisen die Schlitze eine gerade, gebogene, sternförmige, kreuzförmige oder hufeisenförmige Form auf. In einer Ausführungsform weisen die Schlitze jeweils eine Gesamtlänge im Bereich von 0,4 mm bis 3,0 mm auf, beispielsweise im Bereich von 0,8 mm bis 2,5 mm, oder im Bereich von 1,0 bis 2,0 mm.

In einer Ausführungsform weist jedes der Abgabeventile ein erstes Material mit jeweils einem Schlitz auf. In einer Ausführungsform weist jedes der Abgabeventile genau einen, d.h. nicht mehr als einen, Schlitz in dem ersten Material auf.

In einer Ausführungsform weisen alle Schlitze der Abgabeventile jeweils im Wesentlichen dieselbe Länge auf. In einer Ausführungsform weisen alle Schlitze eine Länge auf, die um nicht mehr als 25% nach oben oder unten um einen bestimmten Wert abweicht, beispielsweise eine Länge von 1,00 mm +/- 25%, d.h. z.B. 0,75 mm bis 1,25 mm.

In einer Ausführungsform sind die Schlitze eingerichtet, sich durch eine elastische Rückstellkraft des ersten Materials reversibel zu öffnen und schließen. Durch einen Überdruck eines Fluids in dem Kanal können hierbei die Schlitze geöffnet werden, indem das erste Material durch das Fluid bei Überschreiten eines vorbestimmten Grenzdrucks des Fluids auseinandergedrückt wird.

In einer Ausführungsform sind die Schlitze durch abschnittsweise Auftrennung des ersten Materials herstellbar oder hergestellt, ohne dabei Teile des ersten Materials zu entfernen. Hierbei können die Schlitze beispielsweise durch Stanzen mit einer Klinge hergestellt sein. Solche Schlitz sind vollständig geschlossen, sofern kein Druckunterschied über die beiden gegenüberliegenden Seiten des ersten Materials anliegt. Hierdurch kann ein Eindringen von Gewebe oder Flüssigkeit von außen in den Spacer vermieden werden.

In einer Ausführungsform weisen die Schlitze Wände auf, welche sich im geschlossenen Zustand eines Abgabeventils berühren, und sich beim Öffnen eines Abgabeventils voneinander entfernen.

In einer weiteren Ausführungsform weist der Kanal eine Einlassöffnung zur Aufnahme eines Fluids auf, wobei die Einlassöffnung bevorzugt an der Außenoberfläche des Spacers angeordnet ist.

Weiterhin kann der Spacer einen Fluidverbinder aufweisen. Der Fluidverbinder kann zum Anschluss eines Gefäßes oder einer fluidleitenden Verbindung dienen, um eine Flüssigkeit in den Spacer aufzunehmen. Ein Beispiel für einen Fluidverbinder ist ein Spritzenanschluss, z.B. ein Luer-Lock-Anschluss. Über einen solchen Anschluss kann eine handelsübliche Luer-Lock-Spritze flüssigkeitsdicht und fluidleitend mit der Einlassöffnung des Spacers verbunden werden. Der Fluidverbinder kann lösbar der Einlassöffnung verbindbar sein. Der Fluidverbinder kann operativ mit dem Spacer verbindbar sein.

Der Spacer kann weiterhin eine Zuleitung aufweisen, die mit der Einlassöffnung verbindbar oder verbunden ist. Die Zuleitung kann bevorzugt operativ mit der Einlassöffnung verbindbar oder verbunden sein. In einer Ausführungsform weist der Spacer einen Fluidverbinder auf, der über eine Zuleitung mit der Einlassöffnung verbindbar oder verbunden ist. Die Zuleitung kann beispielsweise einen Schlauch aus einem medizinisch verträglichen Material aufweisen.

In einer Ausführungsform kann der Spacer ein Rückschlagventil aufweisen. Das Rückschlagventil ist bevorzugt eingerichtet, den Austritt eines Fluids aus dem Spacer durch die Einlassöffnung zu verhindern. Das Rückschlagventil kann operativ mit der Einlassöffnung verbindbar oder verbunden sein. Das Rückschlagventil kann unmittelbar an der Einlassöffnung angeordnet sein, oder kann an der Zuleitung oder dem Fluidverbinder angeordnet sein. Somit kann durch die Einlassöffnung ein Fluid in den Spacer eingebracht werden, ohne dass ein Rückfluss des Fluids durch die Einlassöffnung erfolgt.

Zur sicheren Verbindung der Zuleitung mit der Einlassöffnung kann der Spacer weiterhin einen Rastmechanismus aufweisen, der bevorzugt an der Einlassöffnung angeordnet ist. In einer Ausführungsform weist der Rastmechanismus beispielsweise ein oder mehrere bewegliche Rastelemente auf, die ausgebildet und eingerichtet sind, sich radial nach innen zu bewegen, wenn die Zuleitung in die Einlassöffnung eingeführt wird, und in entsprechende Rillen oder Vertiefungen an der Einlassöffnung eingreifen. Der Rastmechanismus kann bevorzugt federbelastetet sein, um ein Eingreifen der Rastelemente an der Einlassöffnung zu ermöglichen.

Der Rastmechanismus kann eine Entriegelung aufweisen, welche zum Lösen der Rastelemente ausgebildet und eingerichtet ist.

In einer weiteren Ausführungsform weist der Spacer weiterhin einen Trokar auf. Ein Trokar weist ein spitzes Ende auf, welches die Durchdringung eines Gewebes erlaubt. Weiterhin weist ein Trokar einen Schaft auf, an dem der Trokar geführt werden kann. Der Schaft kann einen zylinderförmigen Hohlraum aufweisen, ähnlich einer Kanüle.

Der Trokar ist bevorzugt mit der hierin beschriebenen Zuleitung verbindbar. Bevorzugt ist der Trokar lösbar mit einem distalen Ende der Zuleitung verbindbar. Mithilfe des Trokars kann ein medizinischer Anwender die Zuleitung in Patientengewebe einführen und hierdurch an einem gewünschten Ort positionieren und fixieren. Ein Trokar kann die schonende und präzise Durchdringung eines Zielgewebes ermöglichen. Hierbei kann ein Kanal zum gewünschten Verabreichungsort geschaffen werden, wodurch sich der Spacer zur Verabreichung eines medizinischen Fluids entsprechend positionieren lässt. Die Zuleitung kann hierbei durch den mithilfe des Trokars geschaffenen Kanals in ein Patientengewebe eingebracht werden. Bevorzugt ist der Trokar abnehmbar, um nachfolgend an die der Zuleitung mithilfe des Trokars die Aufnahme eines medizinischen Fluids über die Zuleitung zu ermöglichen. Hierbei kann beispielsweise der Trokar von der Zuleitung entfernt und durch einen Fluidverbinder, beispielsweise einen Luer-Lock-Anschluss, ersetzt werden. Danach kann beispielsweise mithilfe einer Luer-Lock-Spritze ein medizinisches Fluid in die Zuleitung gegeben werden, um es mithilfe der Vorrichtung an einem Zielort des Patientengewebes abzugeben.

In einer Ausführungsform kann die hierin beschriebene Einlassöffnung in identischer Weise wie die hierin beschriebenen Aufnahmen ausgestaltet sein. In einer Ausführungsform ist die Einlassöffnung in anderer Weise als die hierin beschriebenen Aufnahmen ausgestaltet.

Der Spacer kann für verschiedene Einsatzorte angepasst sein. In einer Ausführungsform ist der Spacer ausgewählt aus der Gruppe bestehend aus einem Kniegelenkspacer, einem Hüftgelenkspacer, einem Wirbelkörperspacer und einem Marknagelspacer.

Der Kniegelenkspacer kann eine einteiliger oder mehrteiliger Kniegelenkspacer sein. Der Hüftgelenkspacer kann ein einteiliger oder mehrteiliger Hüftgelenkspacer sein. Der Wirbelkörperspacer kann ein einteiliger oder zweiteiliger Wirbelkörperspacer sein. Die hierin beschriebenen Abgabesysteme können bei den mehrteiligen Spacern jeweils entweder in nur einem der beiden Teile, oder in beiden Teilen vorhanden sein.

Die hierher einen beschriebenen Spacer, beispielsweise die Hüftgelenkspacer oder die Kniegelenkspacer, können jeweils ein erstes Teilelement und ein zweites Teilelement aufweisen. Das erste Teilelement und/oder das zweite Teilelement kann zum Einbringen in einen Patientenknochen vorgesehen sein. Hierbei kann eine Verankerung mit Knochenzement erfolgen.

In einer Ausführungsform weisen sowohl das erste Teilelement als auch das zweite Teilelement Abgabeventile auf.

Der Spacer weist in einer Ausführungsform weiterhin einen Zementierbereich auf, welcher keine Einlassöffnungen, Abgabeventile oder Einlassöffnungen aufweist. Dieser Zementierbereich ist bevorzugt dazu ausgestaltet und eingerichtet, durch Verwendung eines Knochenzements im Knochen eines zu behandelnden Patienten befestigt zu werden. Auf diese Weise kann der Spacer nach der Implantation an einem gewünschten Zielort in Position gehalten werden.

Weiterhin ist es möglich, die hierin beschriebene Erfindung als Implantat auszugestalten, das zum dauerhaften Verbleib in einem Patienten vorgesehen ist. Beispielsweise kann eine Osteosyntheseplatte wie hierin beschrieben mit einem Kanal und den hierin beschriebenen Abgabeventilen versehen sein. Demnach stellt die Erfindung in einem Aspekt auch ein Implantat zur Abgabe eines medizinischen Fluids an einen Patienten bereit, wobei das Implantat eine Außenoberfläche und eine an der Außenoberfläche angeordnete Vielzahl von Abgabeventilen aufweist, wobei die Abgabeventile jeweils durch einen innerhalb des Implantats angeordneten Kanal miteinander fluidleitend verbunden sind, und wobei die Abgabeventile ausgestaltet und eingerichtet sind, sich abhängig vom Druck eines Fluids innerhalb des Kanals reversibel zu öffnen, um das Fluid aus den Abgabeventilen abzugeben. Das Implantat kann als Gelenkimplantat ausgestaltet sein, beispielsweise als Hüftgelenksimplantat, Schultergelenksimplantat, oder als Kniegelenksimplantat.

In einer weiteren Ausführungsform ist der hierin beschriebene Spacer oder das hierin beschriebene Implantat ausgebildet und eingerichtet, aus jedem der Abgabeventile gleichzeitig eine im Wesentlichen identische Menge eines Fluids abzugeben. Eine im wesentlichen identische Menge kann beispielsweise eine Menge sein, die eine statistische Standardabweichung von weniger als 10% des arithmetischen Mittelwerts aufweist.

In einer weiteren Ausführungsform ist der hierin beschriebene Spacer oder das hierin beschriebene Implantat ausgebildet und eingerichtet, alle Abgabeventile jeweils gleichzeitig zu öffnen, und/oder jeweils gleichzeitig zu schließen. Abhängig von dem im Kanal anliegenden Druck eines Fluids werden die Abgabeventile jeweils demnach entweder geöffnet oder geschlossen, wie hierin beschrieben.

In einer Ausführungsform sind die Abgabeventile jeweils derart angeordnet, dass sie mit der Außenoberfläche des Spacers abschließen. Durch eine solche Ausgestaltung kann verhindert werden, dass die Abgabeventile in Bezug auf den Spacer Vorsprünge bilden, welche zu einer Verletzung oder Reizung von Patientengewebe führen könnten.

In einer Ausführungsform der Erfindung weist der Kanal mehrere Verzweigungen auf. Hierbei weist bevorzugt jede Verzweigung ein Abgabeventil oder eine Einlassöffnung gemäß der hierin beschriebenen Ausführungsformen auf. Hiermit ist insbesondere gemeint, dass im Falle einer Verzweigung an jedem Seitenarm des Kanals ein Abgabeventil oder eine Einlassöffnung vorhanden ist, wobei die Abgabeventile oder Einlassöffnungen dabei jeweils bevorzugt an der Außenoberfläche des Spacers angeordnet sind.

In einer Ausführungsform sind die Abgabeventile ausgestaltet und eingerichtet, Flüssigkeit nur unidirektional durch die Abgabeventile passieren zu lassen. Dies bedeutet, dass die Abgabeventile in diesem Fall als Ein-Wege-Ventile ausgestaltet sind. Erfindungsgemäß wird hierunter verstanden, dass durch derartige Ventile keine Flüssigkeit von außen durch ein Abgabeventil in den Kanal gelangen kann, solange der Druck eines Fluids in dem Kanal gleich hoch, oder höher, wie der Umgebungsdruck auf der Außenseite des Spacers ist.

In einer Ausführungsform weist der Spacer oder das Implantat ein Gelenkelement auf. Das Gelenkelement kann einen Gelenkkörper aufweisen. Das Gelenkelement kann das erste Teilelement beweglich mit dem zweiten Teilelement verbinden. Der Kanal kann sich aus dem ersten Teilelement durch das Gelenkelement hindurch in das zweite Teilelement erstrecken. Hierbei kann der Kanal entlang einer zentralen Achse des Gelenkelements angeordnet sein. In einer Ausführungsform definiert der Kanal entlang seiner Erstreckungsrichtung aus dem ersten Teilelement durch das Gelenkelement eine Verbindungsachse. Diese Achse ist in einem Zustand definiert, in welchem keine äußere Kraft auf den Spacer einwirkt. Das Gelenkelement kann ausgebildet und eingerichtet sein, eine Bewegung des zweiten Teilelements um mindestens 20° im Winkel zu dieser Verbindungsachse zu erlauben. In einer Ausführungsform beträgt dieser Winkel mindestens 30° oder mindestens 40°. Beispielsweise kann das zweite Teilelement relativ zu dem ersten Teilelement mindestens 20°, mindestens 30° oder mindestens 40° seitlich biegbar sein. Um eine solche Bewegung zu erlauben, kann der Gelenkkörper ein gummielastisches zweites Material umfassen. In einer Ausführungsform besteht der Gelenkkörper aus dem zweiten Material. Das zweite Material kann mit dem hierin beschriebenen ersten Material identisch sein, oder das zweite Material kann sich von dem hierin beschriebenen ersten Material unterscheiden. Das zweite Material kann eine Shore-A-Härte im Bereich von 15 bis 80 aufweisen. In einer Ausführungsform weist das zweite Material eine Shore-A-Härte im Bereich von 20 bis 70 oder im Bereich von 30 bis 60 auf. Das zweite Material kann eine geschlossene Porosität aufweisen.

Der Gelenkkörper kann einen Außendurchmesser aufweisen, welcher zwischen dem 0,25-fachen und dem 0,75-fachen des Außendurchmessers des ihn umgebenden ersten Teilelements oder zweiten Teilelements beträgt. Diese beiden Außendurchmesser sind bevorzugt jeweils in einer Richtung definiert, welche orthogonal zur oben beschriebenen Verbindungsachse liegt. Bevorzugt sind diese beiden Außendurchmesser jeweils entlang derselben Linie definiert.

In einer Ausführungsform weist der Kanal einen Durchmesser auf, der ein Verhältnis zu einem Außendurchmesser des Gelenkkörpers aufweist, welches in einem Bereich von [1:1] bis [1:6] liegt. Der Durchmesser des Kanals ist dabei als lichte Weite des Kanals definiert.

In einer Ausführungsform weist der Spacer eine Verankerung auf, welche den Gelenkkörper mit dem ersten Teilelement und/oder mit dem zweiten Teilelement verbindet. In einer Ausführungsform verbindet die Verankerung den Gelenkkörper unmittelbar mit dem ersten Teilelement oder mit dem zweiten Teilelement. In einer Ausführungsform verbindet die Verankerung den Gelenkkörper mit dem ersten Teilelement. In einer Ausführungsform verbindet die Verankerung den Gelenkkörper mit dem zweiten Teilelement. In einer Ausführungsform verbindet eine erste Verankerung einen ersten Gelenkkörper mit dem ersten Teilelement, und eine zweite Verankerung verbindet einen zweiten Gelenkkörper mit dem zweiten Teilelement. In einer Ausführungsform erstreckt sich der Kanal durch die Verankerung hindurch. In einer Ausführungsform erstreckt sich der Kanal durch den Gelenkkörper hindurch. In einer Ausführungsform erstreckt sich der Kanal durch die Verankerung und durch den Gelenkkörper hindurch. In einer Ausführungsform weist die Verankerung eine im wesentlichen zylindrische Form auf. Die Verankerung kann beispielsweise als Schraube oder Bolzen ausgestaltet sein. Demnach kann die Verankerung ein Gewinde umfassen. Die Verankerung kann auch umlaufende Rillen, Vorsprünge oder sonstige Rastmittel umfassen. Die Verankerung kann einen Hohlraum umfassen, um wie hierin beschrieben einen Teil des Kanals zu bilden.

Die Verankerung kann formschlüssig, kraftschlüssig und/oder stoffschlüssig mit dem zweiten Material verbunden sein. Die Verankerung kann formschlüssig, kraftschlüssig und/oder stoffschlüssig mit dem ersten Teilelement verbunden sein. Die Verankerung kann formschlüssig, kraftschlüssig und/oder stoffschlüssig mit dem zweiten Teilelement verbunden sein.

In einer Ausführungsform kann das Gelenkelement, bevorzugt der Gelenkkörper des Gelenkelements, ein hierin beschriebenes Abgabeventil aufweisen. Das Abgabeventil kann hierbei in dem zweiten Material angeordnet sein. Das zweite Material kann einen Schlitz aufweisen. In einer Ausführungsform ist dieser Schlitz eingerichtet, sich durch eine elastische Rückstellkraft des zweiten Materials reversibel zu öffnen und schließen.

In einer Ausführungsform weist der Spacer mehrere Gelenkelemente auf. Die Gelenkelemente können mittelbar oder unmittelbar miteinander verbunden sein. Beispielsweise können mehrere Gelenkkörper unmittelbar miteinander verbunden sein. Hierzu können die Gelenkkörper beispielsweise Gewinde oder Rastelemente, wie z.B. Vertiefungen und/oder Vorsprünge, aufweisen. Die Gelenkkörper können durch dazwischen liegende Verankerungen, wie hierin beschrieben, miteinander verbunden sein.

Der Spacer kann zusätzlich zu dem ersten Teilelement und dem zweiten Teilelement weitere deratige Teilelemente aufweisen, die jeweils durch die hierin beschriebenen Gelenkelemente miteinander verbunden sind. Hierdurch kann eine größere Beweglichkeit des Spacers erzielt werden. Beispielsweise kann der Spacer in einer solchen Ausführungsform gleichzeitig in unterschiedliche Richtungen gekrümmt werden, in ähnlicher Weise, wie dies die Wirbelgelenke in der Wirbelsäule ermöglichen.

Ein weiterer Aspekt der Erfindung betrifft ein Kit zur Herstellung eines hierin beschriebenen Spacers, das mehrere hierin beschriebene Teilelemente und ein oder mehrere hierin beschriebene Gelenkelemente aufweist. In einer Ausführungsform weist das Kit mehrere untereinander austauschbare Teilelemente auf. Diese Teilelemente können unterschiedliche Größen und/oder Geometrien aufweisen, beispielsweise unterschiedliche Längen und/oder Dicken. Hierdurch kann ein medizinischer Anwender den Spacer individuell an einen zu behandelnden Patienten anpassen.

In einer Ausführungsform ist das Kit ausgebildet und eingerichtet, implantierbaren Spacer zur Abgabe eines medizinischen Fluids an einen Patienten herzustellen, wobei der Spacer eine Außenoberfläche und eine an der Außenoberfläche angeordnete Vielzahl von Abgabeventilen aufweist, wobei die Abgabeventile jeweils durch einen innerhalb des Implantats angeordneten Kanal miteinander fluidleitend verbunden sind, und wobei die Abgabeventile ausgestaltet und eingerichtet sind, sich abhängig vom Druck eines Fluids innerhalb des Kanals reversibel zu öffnen, um das Fluid aus den Abgabeventilen abzugeben.

Ein zweiter Aspekt der Erfindung betrifft ein medizinisches Fluid zur Anwendung in einem medizinischen Verfahren, wobei das Verfahren die nachfolgenden Schritte aufweist:
Bereitstellen eines hierin beschriebenen Spacers;
Einbringen eines medizinischen Fluids in den Kanal des Spacers;
Druckbeaufschlagung des medizinischen Fluids in dem Kanal; um die Abgabeventile druckabhängig zu öffnen und das Fluid an einen Patienten abzugeben.

Das medizinische Verfahren umfasst bevorzugt die Behandlung einer Entzündung, mechanisch bedingten Verletzung (Trauma), Infektion oder onkologischen Erkrankung. Das Verfahren umfasst bevorzugt die Behandlung eines erkrankten Knochens oder Gelenks. Bei der Infektion kann es sich beispielsweise um Osteomyelitis oder Osteitis handeln. Weiterhin kann das Verfahren die Behandlung von Schmerzen umfassen.

In einer Ausführungsform umfasst das medizinische Verfahren eine Gelenkinfektion, beispielsweise eine Hüftgelenksinfektion, Kniegelenksinfektionen, Schultergelenksinfektionen oder Spondylodiszitis. In einer Ausführungsform ist die behandelte Gelenkinfektion operativ bedingt, beispielsweise aufgrund eines chirurgischen Eingriffs zur Implantation eines künstlichen Gelenks. In einigen Ausführungsformen wird eine Gelenkinfektion mithilfe eines medizinischen Fluids behandelt, welches ein Antibiotikum und/oder ein Antimykotikum umfasst.

Eine weitere Ausführungsform gemäß des zweiten Aspekts der Erfindung betrifft ein medizinisches Fluid zur Anwendung in einem medizinischen Verfahren wie oben beschrieben, wobei das Fluid einen Wirkstoff aufweist. Hierbei können grundsätzlich alle der hierin beschriebenen Wirkstoffe verwendet werden. In einer Ausführungsform umfasst das Fluid einen Wirkstoff, der ausgewählt ist aus der Gruppe bestehend aus einem Antibiotikum, einem Antiphlogistikum, einem Anästhetikum und einem Cytostatikum.

In einer Ausführungsform ist der Wirkstoff ausgewählt aus der Gruppe bestehend aus Gentamicin, Tobramycin, Amikacin, Clindamycin, Daptomycin, Vancomycin, Teicoplanin, Dalbavancin, Fosfomycin, Linezolid, Eperezolid, Colistin, Meropenem, Fluconazol, Micafungin, Caspofungin, Metronidazol, Moxifloxazin, Ofloxazin, Levofloxacin, Ciprofloxazin, Rifamycin und Rifampicin.

In einer Ausführungsform ist das medizinische Fluid eine wässrige Lösung eines hierin beschriebenen Wirkstoffs.

In einer Ausführungsform umfasst das medizinische Verfahren die Implantation des Spacers in ein Patientengewebe, bevorzugt im Bereich eines Gelenks oder am Ort einer Fraktur. In einer Ausführungsform umfasst das medizinische Verfahren die Durchdringung von Patientengewebe mithilfe eines Trokars, um eine Zuleitung des Spacers aus dem Körperinneren eines Patienten durch die Haut nach außen zu führen.

In einer Ausführungsform wird das medizinische Fluid mithilfe einer Pumpe in den Kanal des Spacers eingebracht, beispielsweise mithilfe einer Spritzenpumpe. In einer Ausführungsform wird das medizinische Fluid kontinuierlich über einen Zeitraum von mehreren Minuten, mehreren Stunden oder mehreren Tagen an den Patienten abgegeben. In einer Ausführungsform wird das medizinische Fluid wiederholt an den Patienten abgegeben. Beispielsweise kann das medizinische Fluid einmal täglich oder mehrmals täglich, beispielsweise zweimal dreimal oder mehr als dreimal täglich, an den Patienten abgegeben werden. In einer Ausführungsform wird das medizinische Fluid abhängig von einem Gesundheitsparameter des Patienten abgegeben. Der Gesundheitsparameter kann beispielsweise ein diagnostischer Messwert oder der Schmerzzustand des Patienten sein.

In einer Ausführungsform wird das medizinische Fluid mithilfe einer manuellen oder automatischen Steuerung dosiert.

Ein weiterer Aspekt der Erfindung betrifft ein medizinisches Behandlungsverfahren, wobei das Verfahren die nachfolgenden Schritte aufweist:
Bereitstellen eines hierin beschriebenen Spacers, Implantats oder Kits,
Einbringen eines medizinischen Fluids in den Kanal des Spacers oder Implantats, Druckbeaufschlagung des medizinischen Fluids in dem Kanal, um die Abgabeventile druckabhängig zu öffnen und das Fluid an einen Patienten abzugeben.

Die obigen Ausführungen betreffend ein medizinisches Fluid zur Anwendung in einem medizinischen Verfahren gelten hierzu entsprechend.

### FIGUREN

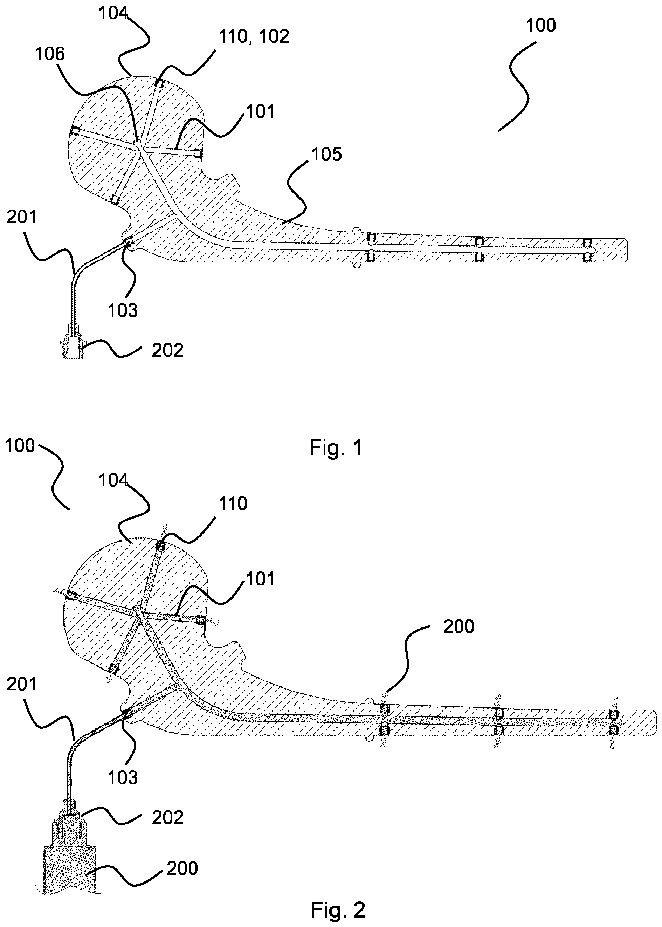

**Figur 1** zeigt einen Querschnitt einer ersten Ausführungsform eines erfindungsgemäßen Spacers **100.** Innerhalb des Spacers ist ein fluidleitender Kanal **101** angeordnet. Der Kanal **101** weist Verzweigungen **106** auf. Der Spacer weist eine Außenoberfläche **104** auf, an welcher Aufnahmen **102** angeordnet sind. In die Aufnahmen **102** sind Abgabeventile **110** eingebracht. Eine der Aufnahmen **102** ist im hier gezeigten Beispiel als Einlassöffnung **103** ausgestaltet, um eine fluidleitende Verbindung mit einer Zuleitung **201** herzustellen. Dabei greift ein proximales Ende der Zuleitung **201** formschlüssig oder kraftschlüssig in die Einlassöffnung **103** ein. Die Zuleitung **201** weist ein distales Ende auf, welches fluidleitend mit einem Fluidverbinder **202** verbunden ist. Der Fluidverbinder **202** ist hier als Luer-Lock-Verbinder ausgestaltet. Die Abgabeventile **110** sind an unterschiedlichen Positionen auf der Außenoberfläche **104** des Spacers angeordnet, um eine möglichst gleichmäßig verteilte Abgabe eines medizinischen Fluids an die Umgebung des Spacers zu ermöglichen. Der Kanal **101** verbindet die Einlassöffnung **103** mit den Abgabeventilen **110,** sodass ein über die Einlassöffnung 103 aufgenommenes Fluid über den Kanal **101** zu den Abgabeventilen **110** gelangen kann. Der Spacer weist weiterhin einen Zementierbereich **105** auf, welcher keine Einlassöffnungen, Abgabeventile oder Einlassöffnungen aufweist. Dieser Zementierbereich **105** ist dazu ausgestaltet und eingerichtet, durch Verwendung eines Knochenzements im Knochen eines zu behandelnden Patienten befestigt zu werden. Auf diese Weise kann der Spacer nach der Implantation an einem gewünschten Zielort in Position gehalten werden.
**Figur 2** zeigt einen Querschnitt eines Spacers gemäß Figur 1, welcher mit einem medizinischen Fluid **200** befüllt ist. Durch die hier gezeigte Ausgestaltung des Spacers kann mithilfe einer Spritze ein medizinisches Fluid über einen Fluidverbinder **202,** eine Zuleitung **201** und eine Einlassöffnung **103** in den Kanal **101** eingefüllt werden. Aufgrund einer Druckbeaufschlagung des Fluids **200** innerhalb des Kanals **101** öffnen sich die Abgabeventile **110,** um das Fluid aus dem Spacer **100** an ein Zielgewebe eines Patienten abzugeben. Die Abgabeventile **110** sind ausgebildet und eingerichtet, sich oberhalb eines vorbestimmten Drucks des Fluids **200** in dem Kanal **101** zu öffnen. Hierdurch kann eine zeitlich und quantitativ gleichmäßige Abgabe des Fluids **200** über alle Abgabeventile **110** erreicht werden.

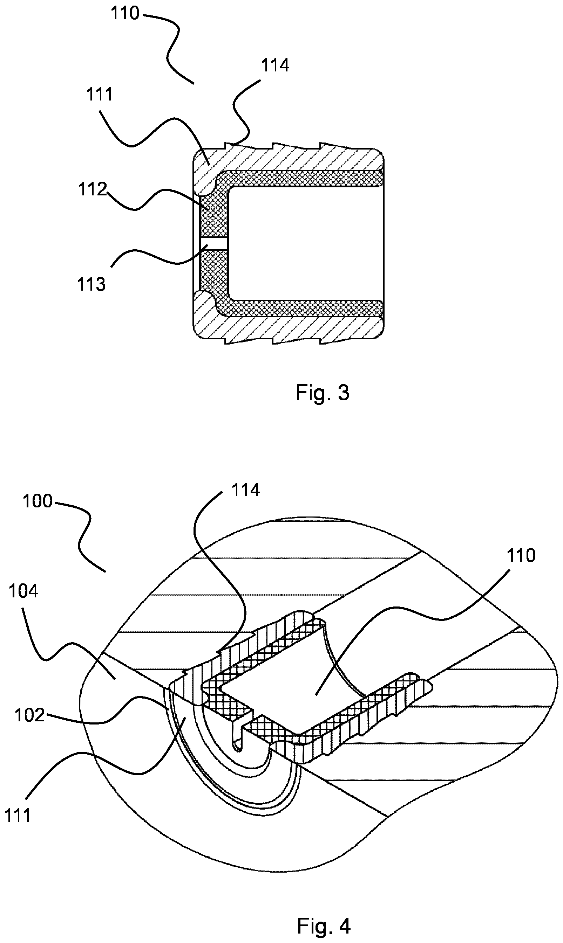

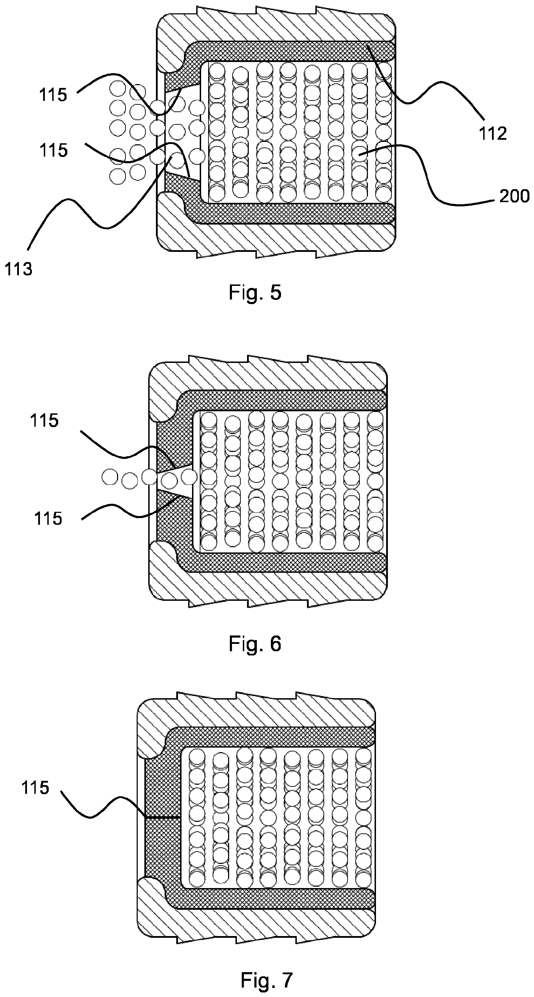

**Figur 3** zeigt eine Ausführungsform eines Abgabeventils **110** in einer Querschnittsansicht. Diese Ausführungsform eines Abgabeventils **110** ist in modularer Bauweise konstruiert, sodass es getrennt hergestellt und in einen erfindungsgemäßen Spacer **100** eingebracht werden kann. Das Abgabeventil **110** weist ein Gehäuse **111** auf, wobei das Gehäuse **111** Vorsprünge **114** aufweist, die zur festen Verbindung mit einer Aufnahme des Spacers ausgebildet sind. In einem Innenraum des Abgabeventils ist ein gummielastisches erstes Material **112** angeordnet, welches einen Schlitz **113** aufweist. Der Schlitz **113** ist ausgebildet und eingerichtet, sich druckabhängig zu öffnen oder zu schließen, wie in den nachfolgenden Figuren 5 bis 7 ausführlicher dargestellt. Der Schlitz **113** ist hier in einem teilweise geöffneten Zustand dargestellt.
**Figur 4** zeigt einen Ausschnitt einer Ausführungsform eines erfindungsgemäßen Spacers **100,** wobei ein modulares Abgabeventil **110** kraftschlüssig in eine Aufnahme **102** des Spacers eingreift. Das Abgabeventil **110** ist dabei derart angeordnet, dass das Gehäuse **111** des Abgabeventils **110** mit der Außenoberfläche **104** des Spacers abschließt. Hierdurch wird verhindert, dass durch die Anordnung des Abgabeventils **110** in der Aufnahme **102** Vorsprünge gebildet werden, welche eine Verletzungsgefahr für Patienten darstellen könnten. Das Abgabeventil 110 ist hier in einem teilweise geöffneten Zustand dargestellt.
**Figur 5** zeigt eine Ausführungsform eines Abgabeventils **110** in einem vollständig geöffneten Zustand. Aufgrund eines erhöhten Drucks des Fluids **200,** welches aus einem Kanal des Spacers in das Abgabeventil **110** fließt, öffnet sich ein Schlitz **113,** der in einem gummielastischen ersten Material **112** angeordnet ist. Der Schlitz **113** weist Schlitzwände auf, die sich durch den erhöhten Druck des Fluids **200** voneinander trennen und voneinander wegbewegen, sodass sich der Schlitz **113** öffnet und eine Abgabe des Fluids **200** aus dem Abgabeventil **110** ermöglicht.
**Figur 6** zeigt eine Ausführungsform eines Abgabeventils **110** in einem teilweise geöffneten Zustand. Der Druck des Fluids **200** ist in der hier gezeigten Zeichnung nur geringfügig oberhalb des Grenzdrucks, bei dem sich der Schlitz **113** öffnet. Die beiden Schlitzwände **115** befinden sich nahe aneinander, und erlauben im Vergleich zu dem in Figur **5** gezeigten Zustand nur eine verhältnismäßig geringe Abgabemenge und Abgabegeschwindigkeit des Fluids **200** aus dem Abgabeventil **110.**
**Figur 7** zeigt eine Ausführungsform eines Abgabeventils **110** in einem geschlossenen Zustand. Der Druck des Fluids **200** ist in der hier gezeigten Zeichnung unterhalb des Grenzdrucks, bei dem sich der Schlitz **113** öffnen würde. Die beiden Schlitzwände **115** berühren sich und liegen vollständig aneinander an, sodass der Schlitz **113** geschlossen ist, und keinen Durchfluss des Fluids **200** erlaubt.

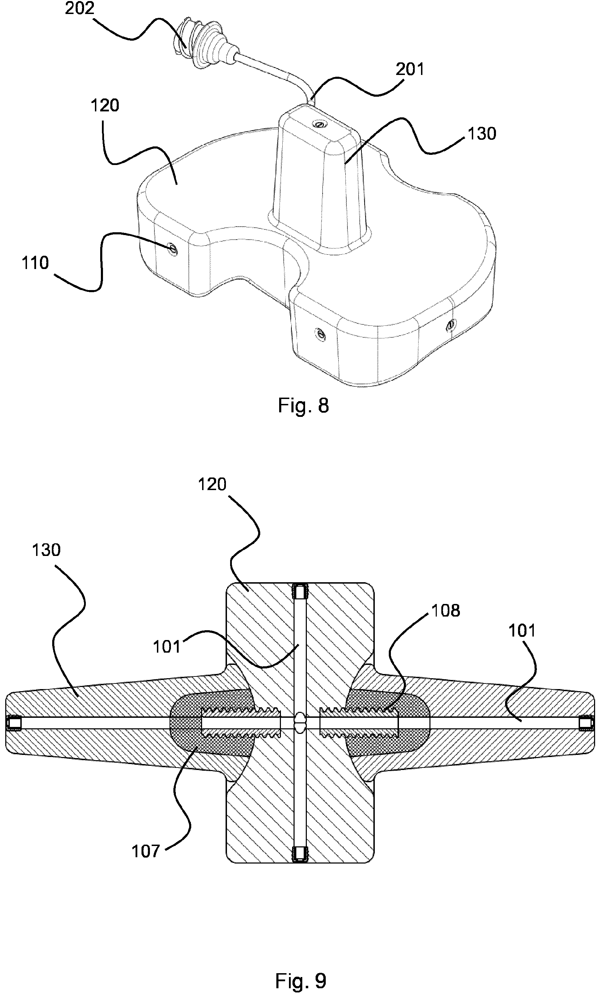

**Figur 8** zeigt eine Ausführungsform eines erfindungsgemäßen Spacers, welche im hier gezeigten Beispiel als Kniegelenkspacer ausgestaltet ist. Der Spacer weist ein erstes Teilelement **120** und ein zweites Teilelement **130** auf, wobei sowohl das erste Teilelement **120** als auch das zweite Teilelement **130** jeweils Abgabeventile **110** aufweisen, die zur Abgabe und Freisetzung eines medizinischen Fluids ausgebildet und eingerichtet sind. Der Spacer weist weiterhin eine Zuleitung **201** mit einem Fluidverbinder **202** zum Einbringen eines medizinischen Fluids auf, wie hierin zuvor beschrieben.
**Figur 9** zeigt eine weitere Ausführungsform eines erfindungsgemäßen Spacers in einer Querschnittsansicht, welcher als Kniegelenkspacer ausgestaltet ist. Diese Ausführungsform weist mehrere Abgabeventile **110** auf, die untereinander und mit einer Einlassöffnung **103** über ein Kanal **101** verbunden sind. Weiterhin umfasst der Spacer in der hier gezeigten Ausführungsform ein Gelenkelement **107,** welches ein gummielastisches Material aufweist. Das Gelenkelement **107** ist formschlüssig mit einem zweiten Teilelement **130** des Spacers verbunden. Weiterhin ist das Gelenkelement **107** formschlüssig und/oder kraftschlüssig über eine Verankerung **108** mit einem ersten Teilelement **120** des Spacers verbunden. Der Kanal **101** erstreckt sich innerhalb des Spacers durch das erste Teilelement **120,** die Verankerung **108,** das Gelenkelement **107** und das zweite Teilelement **130** hindurch. Das Gelenkelement **107** ermöglicht eine seitliche Drehbewegung des zweiten Teilelements **130** gegenüber dem ersten Teilelement **120.** Die hier gezeigte Ausführungsform weist zwei gleichartige zweite Teilelemente **130** auf, die jeweils wie oben beschrieben über eine Verankerung **108** und ein Gelenkelement **107** mit demselben ersten Teilelement **120** verbunden sind.

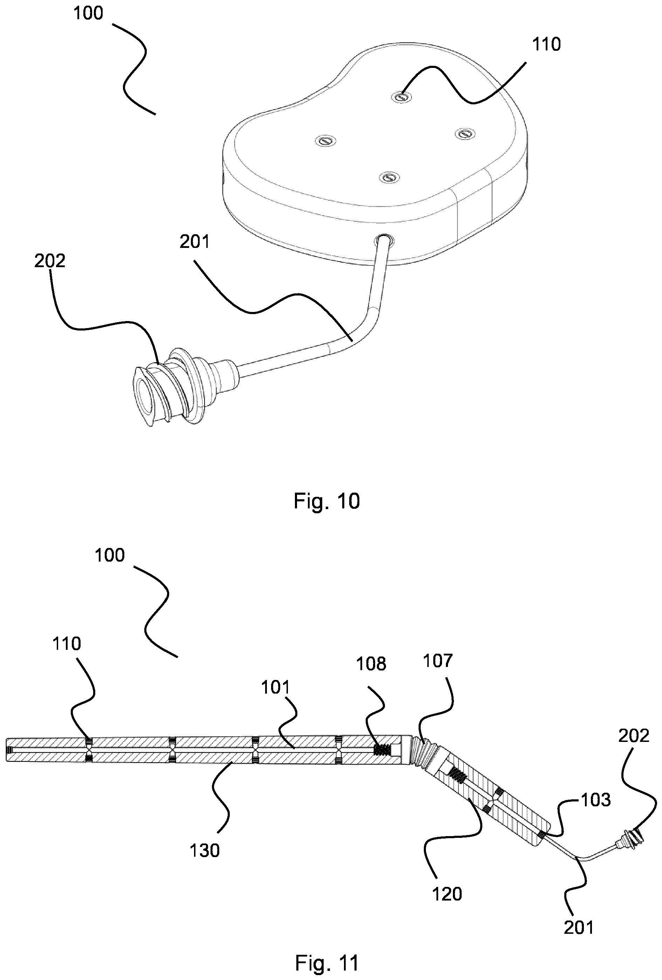

**Figur 10** zeigt eine weitere Ausführungsform eines erfindungsgemäßen Spacers, welcher als Wirbelkörperspacer ausgestaltet ist. Der Spacer **100** weist eine Vielzahl von Abgabeventilen **110,** eine Zuleitung **201** und einem Fluidverbinder **202** auf. Die Zuleitung **201** ist, wie hierin beschrieben, über einen Kanal **101** (hier nicht gezeigt) mit den Abgabeventilen **110** fluidleitend verbunden.
**Figur 11** zeigt eine weitere Ausführungsform eines erfindungsgemäßen Spacers, welcher als Marknagelspacer ausgebildet ist. Der Spacer **100** weist ein erstes Teilelement **120** und ein zweites Teilelement **130** auf, wobei das erste Teilelement **120** über ein Gelenkelement **107** mit dem zweiten Teilelement **130** verbunden ist. Das Gelenkelement **107** kann, ähnlich wie in Figur 9 gezeigt, jeweils mithilfe einer Verankerung **108** mit dem ersten Teilelement **120** und dem zweiten Teilelement **130** verbunden sein. Das erste Teilelement **120** weist eine Einlassöffnung **103** mit einer Zuleitung **201** und einem Fluidverbinder **202** auf, wobei die Einlassöffnung **103** über einen Kanal **101** mit einer Vielzahl von Abgabeventilen **110** fluidleitend verbunden ist, wobei die Abgabeventile **110** sowohl am ersten Teilelement **120** als auch am zweiten Teilelement **130** angeordnet sind. Die Abgabeventile **110** sind gleichmäßig über die Außenoberfläche des Spacers, d.h. über die Außenoberflächen des ersten Teilelements **120** und des zweiten Teilelements **130,** verteilt, um eine möglichst gleichförmige Abgabe eines medizinischen Fluids an die gesamte Umgebung des Spacers **100** zu ermöglichen.

### BEZUGSZEICHENLISTE

- **100**: Spacer
- **101**: Kanal
- **102**: Aufnahme
- **103**: Einlassöffnung
- **104**: Außenoberfläche
- **105**: Zementierbereich
- **106**: Verzweigung
- **107**: Gelenkelement
- **108**: Verankerung
- **110**: Abgabeventil
- **111**: Gehäuse
- **112**: erstes Material
- **113**: Schlitz
- **114**: Vorsprung
- **115**: Schlitzwand
- **120**: erstes Teilelement
- **130**: zweites Teilelement
- **200**: Fluid
- **201**: Zuleitung
- **202**: Fluidverbinder

## Patentansprüche

1. Implantierbarer Spacer **(100)** zur Abgabe eines medizinischen Fluids an einen Patienten, wobei der Spacer eine Außenoberfläche **(104)** und eine an der Außenoberfläche **(104)** angeordnete Vielzahl von Abgabeventilen **(110)** aufweist, wobei die Abgabeventile **(110)** jeweils durch einen innerhalb des Implantats angeordneten Kanal **(101)** miteinander fluidleitend verbunden sind, und wobei die Abgabeventile **(110)** ausgestaltet und eingerichtet sind, sich abhängig vom Druck eines Fluids **(200)** innerhalb des Kanals **(101)** reversibel zu öffnen, um das Fluid **(200)** aus den Abgabeventilen **(110)** abzugeben.

2. Implantierbarer Spacer nach Anspruch 1, wobei die Abgabeventile **(110)** jeweils ein hülsenförmiges Gehäuse (111) aufweisen, und wobei der Spacer eine Vielzahl von Aufnahmen **(102)** aufweist, wobei die Abgabeventile **(110)** durch formschlüssiges und/oder kraftschlüssiges Eingreifen der Gehäuse (111) in die Aufnahmen (102) mit dem Spacer verbindbar sind.

3. Implantierbarer Spacer nach einem der vorangehenden Ansprüche, wobei die Abgabeventile (110) jeweils ein erstes Material (112) aufweisen, wobei das erste Material gummielastisch ist, und das erste Material weiterhin jeweils einen Schlitz (113) aufweist.

4. Implantierbarer Spacer nach Anspruch 3, wobei die Schlitze (113) eingerichtet sind, sich durch eine elastische Rückstellkraft des ersten Materials (112) des Abgabeventils reversibel zu öffnen und schließen.

5. Implantierbarer Spacer nach Anspruch 3 oder 4, wobei das erste Material (112) eine Shore-A-Härte von 30 bis 80, bevorzugt 40 bis 70, weiter bevorzugt 50 bis 60, aufweist.

6. Implantierbarer Spacer nach einem der vorangehenden Ansprüche, wobei der Kanal **(101)** eine Einlassöffnung **(103)** zur Aufnahme eines Fluids aufweist, wobei die Einlassöffnung **(103)** bevorzugt an der Außenoberfläche **(104)** des Spacers angeordnet ist.

7. Implantierbarer Spacer nach Anspruch 6 und Anspruch 2, wobei die Einlassöffnung **(103)** in identischer Weise wie die Aufnahmen **(102)** ausgestaltet ist, oder wobei die Einlassöffnung **(103)** in anderer Weise als die Aufnahmen **(102)** ausgestaltet ist.

8. Implantierbarer Spacer nach einem der vorangehenden Ansprüche, wobei der Spacer aus der Gruppe bestehend aus einem Kniegelenkspacer, einem Hüftgelenkspacer, einem Wirbelkörperspacer und einem Marknagelspacer ausgewählt ist.

9. Implantierbarer Spacer nach einem der vorangehenden Ansprüche, wobei der Spacer ausgebildet und eingerichtet ist, aus jedem der Abgabeventile **(110)** gleichzeitig eine im Wesentlichen identische Menge eines Fluids abzugeben.

10. Implantierbarer Spacer nach einem der vorangehenden Ansprüche, wobei der Spacer ausgebildet und eingerichtet ist, alle Abgabeventile **(110)** jeweils gleichzeitig zu öffnen, und/oder jeweils gleichzeitig zu schließen.

11. Implantierbarer Spacer nach einem der vorangehenden Ansprüche, wobei die Abgabeventile **(110)** jeweils derart angeordnet sind, dass sie mit der Außenoberfläche **(104)** des Spacers abschließen.

12. Implantierbarer Spacer nach einem der vorangehenden Ansprüche, wobei der Kanal **(101)** mehrere Verzweigungen **(106)** aufweist, und wobei bevorzugt jede Verzweigung ein Abgabeventil **(110)** oder eine Einlassöffnung **(103)** gemäß Anspruch 6 aufweist.

13. Implantierbarer Spacer nach einem der vorangehenden Ansprüche, wobei die Abgabeventile **(110)** ausgestaltet und eingerichtet sind, Flüssigkeit nur unidirektional durch die Abgabeventile **(110)** passieren zu lassen.

14. Medizinisches Fluid zur Anwendung in einem medizinischen Verfahren, wobei das Verfahren die nachfolgenden Schritte aufweist:
Bereitstellen eines implantierbaren Spacers **(100)** nach einem der vorangehenden Ansprüche, Einbringen eines medizinischen Fluids in den Kanal **(101)** des Spacers, Druckbeaufschlagung des medizinischen Fluids in dem Kanal **(101),** um die Abgabeventile **(110)** druckabhängig zu öffnen und das Fluid an einen Patienten abzugeben.

15. Medizinisches Fluid zur Anwendung gemäß Anspruch 14, wobei das Fluid einen Wirkstoff aufweist, der ausgewählt ist aus der Gruppe bestehend aus einem Antibiotikum, einem Antiphlogistikum, einem Anästhetikum und einem Cytostatikum.
